(19) Europäisches Patentamt
European Patent Office
Office européen des brevets

(11) **EP 3 187 849 B1**

(12) **EUROPÄISCHE PATENTSCHRIFT**

(45) Veröffentlichungstag und Bekanntmachung des Hinweises auf die Patenterteilung:
**12.12.2018 Patentblatt 2018/50**

(51) Int Cl.:
***G01M 5/00*** *(2006.01)*

(21) Anmeldenummer: **17150226.3**

(22) Anmeldetag: **04.01.2017**

(54) **SYSTEM ZUR BESTIMMUNG EINES BIEGEMOMENTS AN EINEM RUDER UND VERFAHREN ZUR BESTIMMUNG EINER LEISTUNG AN EINEM RUDER**

SYSTEM FOR DETERMINING A BENDING MOMENT ON A RUDDER AND METHOD FOR DETERMINING POWER OUTPUT ON A RUDDER

SYSTEME DE DETERMINATION D'UN MOMENT DE FLEXION SUR UNE RAME ET PROCEDE DE DETERMINATION D'UNE PUISSANCE SUR UNE RAME

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **04.01.2016 EP 16150045**

(43) Veröffentlichungstag der Anmeldung:
**05.07.2017 Patentblatt 2017/27**

(73) Patentinhaber: **Mandanis angewandte Mechanik GmbH**
**6010 Kriens (CH)**

(72) Erfinder:
• **MANDANIS, Georges Marie**
**6010 Kriens (CH)**
• **MANDANIS, Cyrill Alexandre**
**70435 Stuttgart (DE)**

(74) Vertreter: **Liebetanz, Michael**
**Isler & Pedrazzini AG**
**Giesshübelstrasse 45**
**Postfach 1772**
**8027 Zürich (CH)**

(56) Entgegenhaltungen:
GB-A- 2 405 947          US-A1- 2010 167 606
US-A1- 2011 223 816

**Beschreibung**

TECHNISCHES GEBIET

Die vorliegende Erfindung betrifft System zur Bestimmung eines Biegemoments, einer Ruderleistung und ein Verfahren zur Bestimmung einer Ruderleistung an einem Ruder während eines Ruderschlags eines Ruderers im Wasser.

STAND DER TECHNIK

Aus der US 2010/0167606 A1 ist ein Ruder bekannt, bei welchem mittels eines Auslenkungssensors und eines Temperatursensors und eines Prozessors die Kraft ermittelt wird, welche auf ein Ruderblatt während des Ruderns wirkt. Die Sensoren sind in das Innere des Ruders integriert, was eine aufwändige Montage erfordert.
Aus der US 2011/223816 A1 ist ein mehrteiliges Ruder bekannt, bei dem zwischen Ruderblatt und Ruderschaft ein Vorschub erzeugendes angetriebenes Flügelrad vorgesehen ist.
Aus der GB 2,405,947 A ist eine Kraftmesszelle bekannt, die zwischen einem vertikalen Stift eines auf einem Ruderboot angeordneten Stützfusses und einer über Hülsen auf dem vertikalen Stift angeordneten Dolle vorgesehen ist, um auf das Ruder wirkende Kräfte mit Dehnmessstreifen aufnehmen zu können.

DARSTELLUNG DER ERFINDUNG

Es ist daher unter anderem eine Aufgabe der Erfindung, ein Ruder zur Verfügung zu stellen, bei welchem die Montage der Sensoren vereinfacht ist, und welches zuverlässiger, günstiger oder einfacher in der Bedienung ist.
Diese Aufgabe wird durch ein System mit den Merkmalen des Anspruchs 1 gelöst. Dieses System ermöglicht beispielsweise die Überwachung der Ruderschläge beim Training oder beim Wettkampf des Ruderers durch den Ruderer selber oder durch eine Drittperson, wie beispielsweise einen Trainer. Es erlaubt eine detaillierte Analyse des Ruderschlags, mit dem Ziel, diesen zu verbessern. Es erlaubt auch Vergleiche mit gespeicherten Werten, wie mit denen einer früheren Messreihe, beispielsweise eines früheren Trainings oder Rennen oder erlaubt einen Vergleich mit anderen Ruderern, beispielsweise einem Teamkollegen oder einem Weltmeister. Diese Vergleiche helfen dem Ruderer oder dem Trainer, die Leistung des einzelnen Ruderers besser einschätzen zu können und somit ein gezieltes Training zusammenzustellen. Auch für Freizeitruderer ist das System geeignet, da es die eigene Leistung und somit die Entwicklung erkennbar macht.
In einer bevorzugten Ausführungsform weist das System zur Bestimmung eines Biegemoments an einem Ruder während eines Ruderschlags eines Ruderers im Wasser einen Rudergriff, einen Ruderschaft, welcher sich vom Rudergriff erstreckt, ein Ruderblatt, welches am Ruderschaft auf der dem Rudergriff gegenüberliegenden Seite angeordnet ist, und mindestens einen Dehnungsmessstreifen (hier auch kurz als DMS bezeichnet) auf, aus dessen Signal sich das Biegemoment bestimmen lässt, wobei der Ruderschaft mehrteilig ausgebildet ist und wobei zwischen zwei benachbarten Teilen des Ruderschafts eine Messhülse angeordnet ist, welche die beiden benachbarten Teile des Ruderschafts miteinander verbindet und welche den mindestens einen Dehnungsmessstreifen aufweist. Alternativ kann mit dem gleichen System die Kraft auf das Ruderblatt ermittelt werden. Die Schlagzahl des Ruderers, d.h. die Anzahl Ruderschläge über die Zeit lässt sich ebenfalls mit dem System ermitteln.
Diese Bauweise hat unter anderem den Vorteil, dass sich die Messsensoren einfach in das Ruder integrieren lassen und sich die Montage dadurch wesentlich vereinfacht.
Der die Messaufnahme darstellende Mittelteil wird teilweise als Hülse bezeichnet, er kann auch als Messbüchse bezeichnet werden. Die Büchse steht zwischen dem rudergriffseitigen Teil und dem ruderblattseitigen Teil, sie beinhaltet Sensoren und alle Systeme, die zur Sendung der Messsignale (Biegemoment über die Zeit und Rotationsgeschwindigkeit über die Zeit) an eine Auswerteeinheit nötig sind. Die Erfindung beruht inter alia darauf, dass für die Bestimmung der Ruderleistung das Ruder in zwei Stücke getrennt wird, nämlich in den ruderblattseitigen Teil und den rudergriffseitigen Teil, und zwischen ihnen eine Messbüchse installiert, um zwei Messwerte zu ermitteln, nämlich Biegemoment und Rotationsgeschwindigkeit; beides über die Zeit gemessen, um die gewünschte Grösse der Ruderleistung festzustellen. Dabei ist für die Feststellung des Wertes wesentlich, dass man über Zeitintervalle eines Ruderschlags arbeitet und sich nicht für die Kraft allein oder das Moment interessiert. Denn Moment und Rotationsgeschwindigkeit werden dafür parallel über die Zeit am gleichen Ort, nämlich in der Messbüchse gemessen.
Vorzugsweise ist die Messhülse im Wesentlichen zylindrisch mit einer Mittelachse ausgebildet und weist einen Mittelteil und zwei Anschlussteile zur Verbindung mit den beiden benachbarten Teilen des Ruderschafts auf, wobei die Anschlussteile auf sich gegenüberliegenden Seiten des Mittelteils angeordnet sind. Da der Ruderschaft im Allgemeinen einen im Wesentlichen kreisrunden Querschnitt aufweist, eignet sich eine zylindrische Form als Verbindungselement. Alternativ können Messhülsen mit ovalen oder vieleckigen Querschnitten, wie zum Beispiel rechteckigen, quadratischen oder sechseckigen Querschnitten verwendet werden.

Bevorzugt weist die Messhülse im Mittelteil am Umfang mindestens eine Ausnehmung, beispielsweise in der Form einer Abflachung, zur Aufnahme des mindestens einen Dehnungsmessstreifens auf. Die Ausnehmungen erlauben das Anbringen der Dehnungsmessstreifen auf einer ebenen Fläche und schützen gleichzeitig die Dehnungsmessstreifen vor mechanischen Einflüssen, da die Dehnungsmessstreifen im Querschnitt innerhalb der äusseren Umfangslinie der Messhülse liegen.

Es ist vorteilhaft, wenn die Messhülse im Mittelteil in Richtung der Mittelachse am äusseren Umfang zwei sich gegenüberliegende Ausnehmungen zur Aufnahme von jeweils einem Dehnungsmessstreifen aufweist. Es können auch drei, vier, fünf, sechs oder mehr am Umfang gleichmässig oder ungleichmässig verteilte Ausnehmungen vorgesehen sein, wovon jeder jeweils einen Dehnungsmessstreifen aufnehmen kann. Vorteilhafter Weise sind die zwei sich gegenüberliegenden Ausnehmungen derart ausgebildet und ausgerichtet, dass sie eine Auflagefläche für die Dehnungsmessstreifen aufweisen, welche in einer Ebene liegt, welche im Wesentlichen parallel zu einer Ebene liegt, in welcher sich das Ruderblatt erstreckt. In diesen Auflageflächen können aufgrund der Richtung der Krafteinleitung am Ruderblatt, d.h. im Wesentlichen senkrecht auf das Ruderblatt, die grössten Dehnungen gemessen werden.

Alternativ können die Dehnungsmessstreifen nicht in Ausnehmungen sondern direkt am äusseren Umfang der Messhülse angeordnet sein. Eine weitere Alternative sieht die Anordnung der Dehnungsmessstreifen im Innern der Messhülse vor, wenn diese in ihrem Innern dafür vorgesehenen Ausnehmungen aufweist. Solche innere Ausnehmungen können sich entlang der Längsachse der Messhülse erstrecken und können beispielsweise Bohrungen sein, welche sich von einer Seite gegen das Innere der Messhülse erstrecken. Diese Bohrungen können sich über einen Bereich der Länge der Messhülse erstrecken oder über ihre gesamte Länge.

Im Falle von zwei am Umfang der Messhülse gegenüberliegenden Dehnungsmessstreifen messen diese die lokalen Dehnungen, und die Differenz dieser Dehnungen ist proportional zum Biegemoment, welches durch eine auf das Ruderblatt wirkende Kraft erzeugt wird.

Vorzugsweise sind die beiden Anschlussteile bezüglich des Mittelteils abgesetzt und der Mittelteil bildet einen Anschlag für die beiden benachbarten Teile des Ruderschafts. Auf diese Weise können die Anschlussteile leicht in die benachbarten Teile des Ruderschafts eingeschoben werden und mit diesen verbunden werden. Für eine sichere Verbindung kann ein Gewinde vorhanden sein. Der Anschlag verhindert, dass die Anschlussteile ungenügend in die Ruderschäfte eingeschoben werden. Zudem vergrössert der Anschlag die Fläche der Verbindungsstelle und verbessert somit die Verbindung zwischen Messhülse und Ruderschaft.

Die Messhülse kann eine zentrische Bohrung aufweisen, welche sich entlang der Mittelachse durch die gesamte Messhülse erstreckt. Alternativ kann sie sich nur über eine gewisse Länge entlang der Mittelachse erstrecken, so dass die Messhülse auf der einen Seite verschlossen ist. Die zentrische Bohrung ermöglicht eine interne Führung von Kabeln der Dehnungsmessstreifen zu weiteren elektronischen Bauteilen, ermöglicht die Führung von Kabeln von weiteren elektronischen Bauteilen oder erlaubt die Unterbringung von weiteren elektronischen Bauteilen. Die zentrische Bohrung kann auch zur Aufnahme der Dehnungsmessstreifen dienen. Allerdings verringert sich dann die Empfindlichkeit stark, wenn die Bohrung nicht einen solch grossen Innendurchmesser hat, dass die Messhülse nur noch mit einem dünnen Mantel übrig bleibt.

In einer anderen, bevorzugten Ausführungsform weist die Messaufnahme einen in longitudinaler Richtung der Messaufnahme ausgerichteten, radialen Schlitz auf, in den eine mit dem oder den Dehnungsmessstreifen verbundene Schaltung eingeschoben ist. Dabei kann optional der Schlitz kein Sackloch sein, sondern mindestens eine durchgehende Öffnung aufweisen, um die eingeschobene Schaltung mit einem oder mehreren nahe der Öffnung angeordneten Dehnungsmessstreifen durch die Messaufnahme selber zu verbinden. Vorteilhafterweise wird der Platz dann weiter dadurch genutzt, dass eine Energiequelle neben der Schaltung in den Schlitz eingeschoben ist. Dann kann die Schaltung oder Verarbeitungseinheit auch ein mikroelektromechanisches System oder Beschleunigungssensor aufweisen, welches ausgebildet ist, um die Rotationbewegung des Ruders zu messen und ein entsprechendes Signal zu übermitteln, entweder zusammen mit dem Momentwert oder auch alleine drahtlos an mindestens einen Empfänger.

In einer vorzugsweisen Ausführungsform ist der Ruderschaft zweiteilig und ist in einen ruderblattseitigen Teil und einen rudergriffseitigen Teil aufgeteilt, wobei der ruderblattseitige Teil an seinem dem Ruderblatt gegenüberliegenden Ende eine Manschette mit einem Ring zur Befestigung des Ruders an einem Ruderboot aufweist und wobei die Messhülse anschliessend an die Manschette mit dem ruderblattseitigen Teil verbunden ist. Die Dolle ist dabei das aufnehmende Element des Ruders auf dem Boot. Diese Anordnung ermöglicht eine einfache Montage und Kabel und allfällige elektronische Bauteile lassen sich leicht im rudergriffseitigen Teil unterbringen. Die Anordnung der Messhülse anschliessend an die Manschette mit dem Ring ist zu bevorzugen, da in diesem Bereich die Dehnung des Ruderschafts, bzw. der Messhülse am grössten ist und somit die Dehnungsmessstreifen einen grösseren Signalbereich haben, was zu einer grösseren Genauigkeit der Messresultate führt.

Alternativ ist der Ruderschaft zweiteilig und ist in einen ruderblattseitigen Teil und einen rudergriffseitigen Teil aufgeteilt ist, wobei der rudergriffseitige Teil an seinem dem

Rudergriff gegenüberliegenden Ende eine Manschette mit einem Ring zur Befestigung des Ruders an einem Ruderboot aufweist und wobei die Messhülse anschliessend an die Manschette mit dem rudergriffseitigen Teil verbunden ist.

Alternativ kann der Ruderschaft drei, vier oder mehr Teile aufweisen und eine oder mehrere Messhülsen können zwischen jeweils zwei benachbarten Teilen des Ruderschafts angeordnet werden, um die jeweils zwei benachbarten Teile des Ruderschafts miteinander zu verbinden. Durch die Anordnung von mehreren Messhülsen entlang des Ruderschafts, kann die Dehnung des Ruderschafts über die Länge, bzw. der Messhülsen am jeweiligen Ort ermittelt werden.

Vorzugsweise ist die Messhülse in die beiden benachbarten Teile des Ruderschafts eingeklebt. Diese feste Verbindung ist platzsparend, leicht und zeichnet sich durch eine hohe Festigkeit aus. Alternativ kann die Messhülse mit den benachbarten Teilen lösbar verbunden werden, beispielsweise durch eine Schraub-, Dreh- oder Steckverbindung. Durch eine solche lösbare Verbindung kann das Ruder in mehrere Teile zerlegt werden, wodurch beispielsweise bei der Lagerung weniger Platz benötigt wird.

Bevorzugt weist die Messhülse zwei sich am Umfang gegenüberliegende Dehnungsmessstreifen zur Messung der Dehnung an der Oberfläche der Messhülse auf. Durch diese Anordnung kann auf der einen Seite die Dehnung und auf der gegenüberliegenden Seite die Stauchung des Ruderschafts, bzw. der Messhülse gemessen werden.

Alternativ können drei vier oder mehr Dehnungsmessstreifen verwendet werden, welche in den dafür vorgesehenen Ausnehmungen am Umfang angeordnet werden können. Durch die Anordnung von mehreren Dehnungsmessstreifen am Umfang, kann die Richtung der Krafteinwirkung auf das Ruder ermittelt werden. Bei vier Dehnungsmessstreifen in einem regelmässigen Winkelabstand von 90 Grad, sind bei einem Ruderschlag nur zwei gegenüberliegende im wesentlichen senkrecht (d.h. dem Verschwenken des Ruders folgend) zur Vortriebsrichtung angeordnete Dehnungsmessstreifen unter Zug und Stauchung, während die beiden anderen Dehnungsmessstreifen, abgesehen von einer Neigung des Ruders, im wesentlichen horizontal zur Wasserfläche / Bootsboden angeordnet sind und keine grösseren Kräfte und Momente in ihrer Messrichtung erfahren.

In einer bevorzugten Ausführungsform weist das System ferner eine Verarbeitungseinheit auf, welche durch Kabel mit dem mindestens einen Dehnungsmessstreifen elektrisch leitend verbunden ist, weist mindestens eine Energiequelle auf, welche mit dem mindestens einen Dehnungsmessstreifen und der Verarbeitungseinheit elektrisch leitend verbunden ist, um diese mit elektrischer Energie zu versorgen. Die Verarbeitungseinheit weist einen DMS-Verstärker auf, welcher ausgebildet ist, um ein von dem mindestens einen Dehnungsmessstreifen erzeugtes Signal zu verstärken und drahtlos an mindestens einen Empfänger zu übermitteln, insbesondere eine kabellose Übertragung über Bluetooth zu einem ortsnah angeordneten Gerät. Die Auswertung könnte auch am Ruder selber erfolgen und auch die Darstellung der Ergebnisse am Ruder selber ist möglich. Dann sind Display bzw. Eingabeeinheit am Ruder vorgesehen, beispielsweise könnte auch ein Halter für ein Smartphone am Ruder vorgesehen sein; dies kann als zusätzliche Option angesehen werden. Insofern sind insbesondere kabellose Übertragungen zum Beispiel über Bluetooth oder Kabelverbindungen zu ortsnah angeordneten Geräten mögliche Ausgestaltungen.

Vorzugsweise weist die Verarbeitungseinheit ein mikroelektromechanisches System (MEMS) auf, welches ausgebildet ist, um die Rotationbewegung des Ruders während eines Ruderschlags eines Ruderers im Wasser zu messen und ein entsprechendes Signal zum Beispiel drahtlos an mindestens einen Empfänger zu übermitteln. Es ist aber auch eine Drahtverbindung zum Beispiel zu einem am Ruder befestigbaren Smartphone möglich. Damit wird zur Ermittlung der Ruderleistung neben der Momentmessung auch die notwendige Rotationsmessung in einfacher Weise durchgeführt.

Durch die drahtlose Übermittlung der Signale an einen externen Empfänger, d.h. an ein Gerät, welches nicht fix mit dem Ruder verbunden ist oder in dieses integriert ist, kann ein robustes und gegen mechanische Einflüsse unempfindliches System bereitgestellt werden. Zudem erlaubt ein solches System die flexible Nutzung der zur Verfügung gestellten Signale durch einen oder mehrere Empfänger.

Bevorzugt ist die Verarbeitungseinheit im rudergriffseitigen Teil des Ruderschafts angeordnet. Alternativ kann die Verarbeitungseinheit im ruderblattseitigen Teil des Ruderschafts angeordnet sein.

Vorzugsweise ist der mindestens eine Empfänger ein Desktop, ein Laptop, ein Tablet oder ein Smartphone oder der mindestens eine Empfänger ist eine Einheit, beispielsweise ein USB-Stick, welcher mit einem Desktop, einem Laptop, einem Tablet oder einem Smartphone verbindbar ist.

Bei der Integration des Empfängers im Ruder sowie bei einem vom Ruder im Wesentlichen unabhängigen Empfänger, können die übertragenen Signale nicht nur empfangen und ausgewertet werden, sie können auch für eine spätere Verwendung gespeichert werden. Somit lässt sich die Entwicklung eines Ruderers darstellen und auswerten.

Der entsprechende Empfänger besitzt ein Programm oder eine App, mit welcher die übermittelten Signale genutzt werden können, um beispielsweise die Schlagzahl und/oder die Ruderleistung zu ermitteln, ein Monitoring der ermittelten Daten und andere Vergleichsoperationen zu ermöglichen.

Zum Schutz der Messhülse, bzw. der an der Messhülse angeordneten Dehnungsmessstreifen, kann über dieser, bzw. um diese eine Abdeckung oder Schutzhülse angeordnet sein.

Das mikroelektromechanische System MEMS, der DMS Messverstärker und Sender und die Energiequelle können getrennt von der Hülse vorgesehen sein- In anderen Serien sind aus praktischen und technischen Gründen die drei oben erwähnten Elemente in dem Zylinder der Messaufnahme integriert.

Ein bevorzugtes Verfahren zur Bestimmung einer Leistung an einem Ruder während eines Ruderschlags eines Ruderers im Wasser, weist die folgenden Schritte auf:

Ermitteln des Biegemoments am Ruder über die Zeit mittels mindestens eines Dehnungsmessstreifens, welcher an einer Messhülse angeordnet ist, welche zwischen zwei benachbarten Teilen eines Ruderschafts des Ruders angeordnet ist und diese miteinander verbindet;
Ermitteln der Rotationsbewegung des Ruders um eine Achse parallel zum Biegemomentvektor über die Zeit mittels eines mikroelektromechanischen Systems, welches am Ruder angeordnet ist; und
Ermitteln der Ruderleistung über die Zeit aus dem Produkt des Biegemoments und der Rotationsbewegung.

Die Ermittlung der Rotationsbewegung des Ruders um eine Achse parallel zum Biegemomentvektor über die Zeit kann dabei in Bezug auf die Zeiterfassung bedingen, dass die Zeit gemessen wird, während der die Dehnungsmessstreifen zur Biegemomentbestimmung bei dieser Messung im Wesentlichen parallel zur Dollenachse stehen, während sie bei der Ruderrückbewegung ohne Krafteinwirkung im Wesentlichen senkrecht zur Dollenachse stehen. Dann erfasst das mikroelektromechanische System diese Zug-Zeit und den überstrichenen Winkel und zur Leistungsermittlung wird die Rotationsgeschwindigkeit mit dem Biegemoment multipliziert, wobei es sich um ein skalares Produkt des Betrags der beiden Vektorwerte handelt. Hier wird als Ruderhauptachse die Dollenachse, d.h. die Achse, um welche die Dolle rotiert, angesehen.

Vorzugsweise sind zwei sich am Umfang der Messhülse gegenüberliegende Dehnungsmessstreifen vorhanden, um die lokalen Dehnungen zu ermitteln.

Es gilt die folgende Formel für die Berechnung der Ruderleistung über die Zeit:

$$L(t) = M(t) \cdot \omega(t)$$

falls der Vektor M(t) parallel zu $\omega(t)$ steht. Mit anderen Worten: Dabei ist L(t) das skalare Produkt zwischen den Vektoren M(t) und $\omega(t)$.

Dabei ist M(t) das Biegemoment am Ruder in Newtonmeter über die Zeit ist. Also ist L(t) das skalare Produkt von M(t) // $\omega(t)$ und es gilt:

$$M(t) = A \cdot [DMS\_1(t) - DMS\_2(t)]$$

wobei A ein Proportionalitätsfaktor ist und wobei DMS_1(t) ein Messsignal eines ersten Dehnungsmessstreifen über die Zeit ist und wobei DMS_2(t) ein Messsignal eines zweiten Dehnungsmessstreifen über die Zeit ist, wobei $\omega(t)$ die Winkelgeschwindigkeit der Rotationsbewegung des Ruders über die Zeit ist und es gilt:

$$\omega(t) = d\varphi(t)/dt$$

wobei $\varphi(t)$ der Drehwinkel der Rotationsbewegung des Ruders in rad oder Bogenmass um eine Achse parallel zum Momentvektor über die Zeit ist.

Ein Vorteil der Vorrichtung ist es, dass Temperatureffekte nicht kompensiert werden müssen. Beim Start der Messung wird der Messwert bei einer Ruderkraft "Null" auf "Null" gesetzt. Während der Messung hat sich die Temperatur der Messhülse stabilisiert, so dass keine Temperaturstörung zu befürchten ist. Optional kann man in der Messhülse zusätzliche unbelastete DMS einbauen, zum Beispiel quer zu den oben genannten DMS, so dass der Temperatureffekt vollkommen kompensiert wird.

Weitere Ausführungsformen sind in den abhängigen Ansprüchen angegeben.

KURZE BESCHREIBUNG DER ZEICHNUNGEN

Bevorzugte Ausführungsformen der Erfindung werden im Folgenden anhand der Zeichnungen beschrieben, die lediglich zur Erläuterung dienen und nicht einschränkend auszulegen sind. In den Zeichnungen zeigen:

Fig. 1    eine perspektivische Ansicht eines Systems gemäss einem Ausführungsbeispiel der Erfindung mit einem Ruder und einem Empfänger,

Fig. 2    eine perspektivische Explosionsansicht von Teilen eines Ruderschafts des Ruders und von einer Messhülse der Fig. 1,

Fig. 3    eine Schnittansicht der Messhülse der Fig. 2 mit zwei Dehnungsmessstreifen,

Fig. 4    eine schematische Darstellung einer erfindungsgemässen Verarbeitungseinheit und der damit verbundenen Energiequelle und den damit verbundenen Dehnungsmessstreifen;

Fig. 5    eine perspektivische Ansicht einer Messhülse für ein System gemäss einem weiteren Ausführungsbeispiel der Erfindung;

Fig. 6    eine perspektivische Ansicht der Messhülse nach Fig. 5, die um 180 Grad entlang ihrer Längsachse gedreht ist;

Fig. 7    Messkurven von Moment und Rotationsgeschwindigkeit eines Ruders im praktischen Betrieb einer Messaufnahme nach Fig. 5, und

Fig. 8    eine Zusammenschau einer verkleinerten Version der Fig. 7 mit sich aus einer Auswertung ergebenden Werten von Leistung und geleisteter Arbeit über die dargestellten fünf Ruderschläge.

## BESCHREIBUNG BEVORZUGTER AUSFÜHRUNGSFORMEN

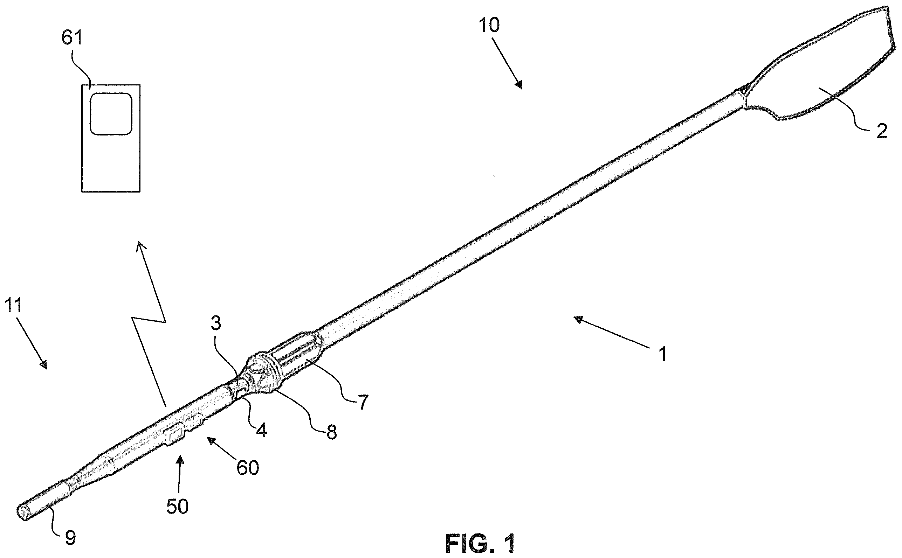

Die Fig. 1 zeigt eine perspektivische Ansicht eines erfindungsgemässen Systems mit einem Ruder und einem Empfänger 61. Das Ruder weist einen Rudergriff 9 und einen Ruderschaft 1 auf, welche sich vom Rudergriff 9 erstreckt. Das Ruder weist ferner ein Ruderblatt 2 auf, welches am Ruderschaft 1 auf der dem Rudergriff 9 gegenüberliegenden Seite angeordnet ist. Der Ruderschaft 1 ist mehrteilig ausgebildet, wobei zwischen zwei benachbarten Teilen des Ruderschafts 1 eine Messhülse 3 angeordnet ist, welche die beiden benachbarten Teile des Ruderschafts 1 miteinander verbindet und welche den mindestens einen Dehnungsmessstreifen 4 aufweist. Der in der Fig. 1 dargestellte Ruderschaft 1 ist zweiteilig und in einen ruderblattseitigen Teil 10 und einen rudergriffseitigen Teil 11 aufgeteilt. Der ruderblattseitige Teil 10 weist an seinem dem Ruderblatt 2 gegenüberliegenden Ende eine Manschette 7 mit einem Ring 8 zur Befestigung des Ruders an einem Ruderboot auf. Die Messhülse 3 ist anschliessend an die Manschette 7 mit dem ruderblattseitigen Teil 10 des Ruderschafts 1 verbunden. Am rudergriffseitigen Teil 11 des Ruderschafts 1 ist eine Verarbeitungseinheit 50 und eine Energiequelle 60 an dessen Aussenseite dargestellt. Diese können jedoch auch im Innern des Ruderschafts 1 angeordnet sein und sind in diesem Falle von aussen nicht sichtbar.

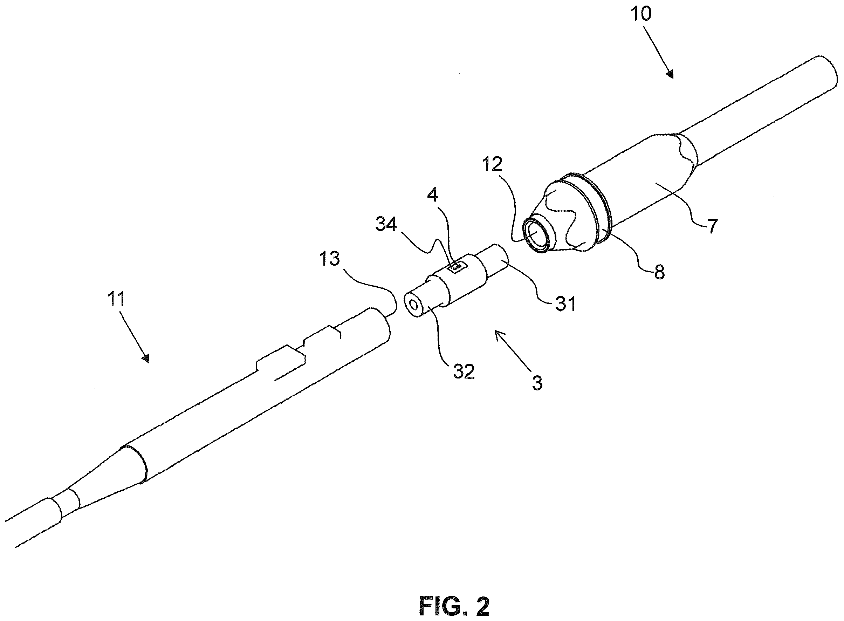

Die Fig. 2 zeigt eine perspektivische Explosionsansicht der Teile des Ruderschafts 1 und von der Messhülse der Fig. 1, wobei die dargestellten Teile und die Messhülse bezüglich der Fig. 1 um 90 Grad bezüglich der Längsachse des Ruderschafts 1 gedreht sind. Die Messhülse 3 ist im Wesentlichen zylindrisch ausgebildet und weist einen Mittelteil 30 und zwei Anschlussteile 31, 32 zur Verbindung mit den beiden benachbarten Teilen des Ruderschafts 1 auf, wobei die Anschlussteile 31, 32 in Bezug auf die Längsrichtung der Messhülse 1 auf sich gegenüberliegenden Seiten des Mittelteils 30 angeordnet sind.

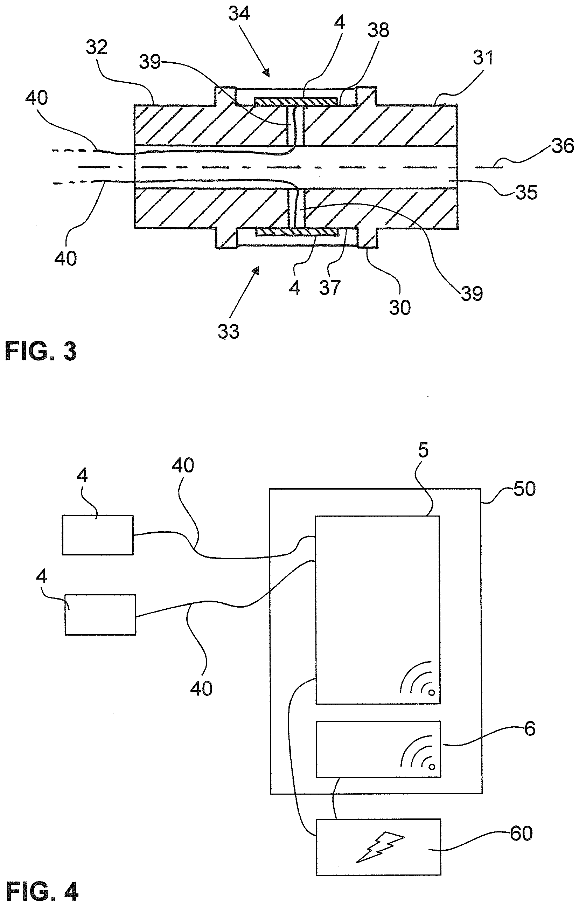

Die Messhülse 3 weist im Mittelteil 30 am Umfang mindestens eine Ausnehmung 34 zur Aufnahme des mindestens einen Dehnungsmessstreifens 4 auf. Bei der Ausnehmung 34 handelt es sich um eine Abflachung des Umfangs des Mittelteils 30, wobei eine ebene Fläche entsteht, welche sich zur Anordnung eines Dehnungsmessstreifen 4 eignet. Auf der bezüglich des Umfangs gegenüberliegenden Seite des Mittelteils 30 ist eine nicht dargestellte, zur Ausnehmung 34 identische Ausnehmung vorgesehen, wie dies in der Fig. 3 dargestellt ist.

Der ruderblattseitige Teil 10 des Ruderschafts 1 weist an seiner dem Ruderblatt 2 gegenüberliegenden Seite eine ruderblattseitige Aufnahme 12 zur Aufnahme eines ersten Anschlussteils 31 der beiden Anschlussteile auf. Der rudergriffseitige Teil 11 des Ruderschafts 1 weist an seiner dem Rudergriff 9 gegenüberliegenden Seite eine nicht dargestellte rudergriffseitige Aufnahme 13 zur Aufnahme eines zweiten Anschlussteils 32 der beiden Anschlussteile auf.

Die Fig. 3 zeigt eine Schnittansicht der Messhülse 3 der Fig. 2 mit zwei Dehnungsmessstreifen 4. Die im Wesentlichen zylindrische Messhülse 3 weist einen Grundkörper auf, der bezüglich der Mittelachse 36 rotationssymmetrisch ist. Die Anschlussteile 31, 32 sind bezüglich des Mittelteils 30 abgesetzt, d.h. die Anschlussteile 31, 32 wiesen im Querschnitt einen geringeren Aussendurchmesser auf als der Mittelteil. Der Mittelteil 30 bildet somit bezüglich der Mittelachse 36 an jedem seiner beiden Enden einen seitlichen Anschlag, an welchem jeweils eines der beiden benachbarten Teile des Ruderschafts 1 anschlagen kann. Die Messhülse 3 weist im Mittelteil 30 in Richtung der Mittelachse 36 am äusseren Umfang zwei sich gegenüberliegende Ausnehmungen 33, 34 zur Aufnahme von jeweils einem Dehnungsmessstreifen 4 auf. Durch die Ausgestaltung der Ausnehmungen entstehen an der Oberfläche der Messhülse ebene Auflageflächen, welche sich am Umfang gegenüberliegen. Eine erste Ausnehmung 33 weist eine erste Auflagefläche 37 auf und eine zweite Ausnehmung 34 weist eine zweite Auflagefläche 38 auf, welche im Wesentlichen parallel zur ersten Auflagefläche 37 ist. Auf den Auflageflächen 37, 38 sind die Dehnungsmessstreifen 4 angeordnet. Die Messhülse 3 weist eine zentrische Bohrung 35 auf, welche sich entlang der Mittelachse 36 durch die gesamte Messhülse 3 hindurch erstreckt. Von den Auflageflächen 37, 38 führen radiale Bohrungen 39 bis zur zentrischen Bohrung 35. Die radialen Bohrungen 39 zusammen mit der zentrischen Bohrung 35 bilden einen Kanal zur Führung von Kabel 40 des jeweiligen Dehnungsmessstreifens 4 in das Schaftinnere.

Die Fig. 4 zeigt eine schematische Darstellung einer erfindungsgemässen Verarbeitungseinheit 50 und der damit verbundenen Energiequelle 60 und den damit verbundenen Dehnungsmessstreifen 4. Die Verarbeitungseinheit 50 weist einem DMS-Verstärker 5 auf, welcher ausgebildet ist, um ein von einem Dehnungsmessstreifen 4 erzeugtes Signal zu verstärken und dieses drahtlos an mindestens einen Empfänger 61 zu übermitteln. Die Verarbeitungseinheit 50 weist ferner ein mikroelektromechanisches System 6 (MEMS) auf, welches ausgebildet ist, um die Rotationbewegung des Ruders zu messen und ein entsprechendes Signal drahtlos an einen Empfänger 61, wie in Fig. 1 dargestellt, zu übermitteln. Wesentlich ist der Einsatz der Beschleunigungssensoren, um die (abgesehen vom Eintauchen und Herausheben) im wesentlichen horizontale Bewegung des Ruders zu erfassen. Die Drehbewegung des Ruders um die Longitudinalachse des Ruders kann, muss aber nicht bei der Messung umfasst sein.

Die Verbindungen zwischen den Dehnungsmessstreifen 4 und dem DMS-Verstärker 5 oder zwischen der Energiequelle 60 und DMS-Verstärker 5 oder zwischen der Energiequelle 60 und dem MEMS 6 sind als Kabel 40 ausgebildet. Für die Momentmessung sind die Dehnungsmessstreifen 4 mit dem Verstärker 5 verbunden, welcher mit dem Sender verbunden ist. Eine Energiequelle speist den Verstärker und den Sender. Die Vorrichtung für die Rotationsmessung ist nach der dargestellten Ausführung total getrennt. In dem kleinen Quader ist das mikroelektromechanische System 6 (gross wie ein USB-Stick) vorgesehen und darin sind das Gyroskop und der Sender eingebaut.

Für die beiden Dehnungsmessstreifen 4 reicht ein DMS-Verstärker 5. Für jeden Dehnungsmessstreifen 4 kann ein DMS-Verstärker 5 vorgesehen sein. Die DMS 4 können auch in einem Stromkreis Type Wheatstone-Brücke geschaltet, sodass man nicht sagen kann, dass jeder DMS einen Verstärker braucht. Der Strom des Stromkreises wird verstärkt.

Das Symbol der drahtlosen Übertragung betrifft beim MEMS 6 wie beim Verstärker 5 die Übermittlung an eine externe Auswerteeinheit, welches beispielsweise ein Smartphone etc. sein kann. Prinzipiell wäre auch eine Auswertung auf einer gedruckten Schaltung am Ruder möglich, dann würden nur Ergebnisse zur (optischen/akustischen) Darstellung übermittelt.

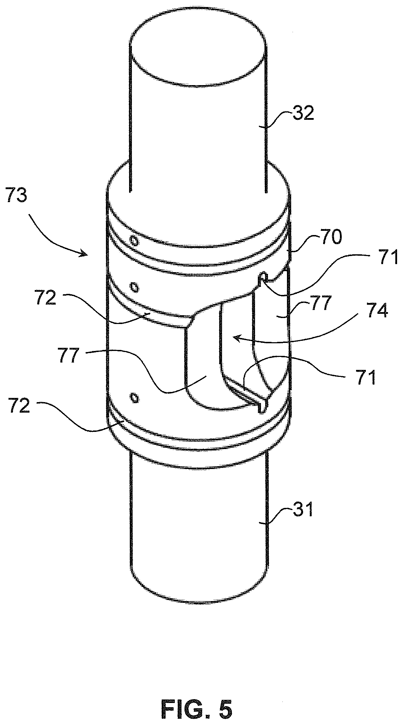

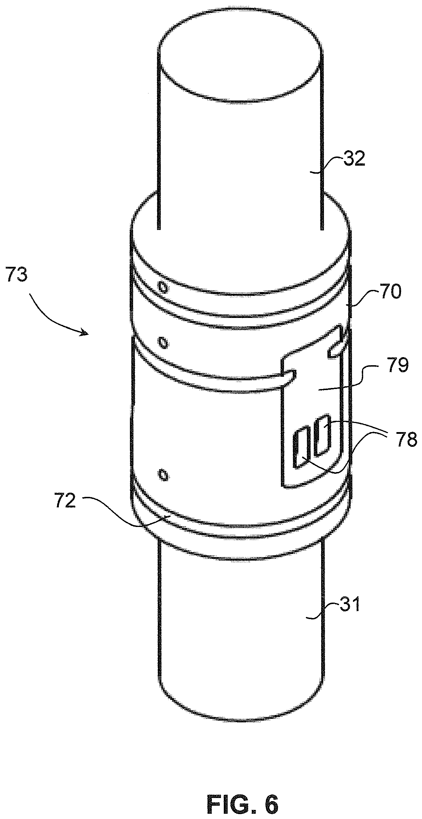

Die Fig. 5 zeigt eine perspektivische Ansicht einer Messaufnahme 73 für ein System gemäss einem weiteren Ausführungsbeispiel der Erfindung, ähnlich zur Messhülse 3 der Fig. 2. Gleiche und ähnliche Merkmale sind in dieser Beschreibung mit gleichen oder ähnlichen Bezugszeichen belegt. Die Fig. 6 zeigt eine weitere perspektivische Ansicht der Messaufnahme 73 nach Fig. 5, die hier um 180 Grad entlang ihrer Längsachse gedreht ist.

Die Messaufnahme 73 verfügt über ein Mittelteil und zwei Anschlussteile 31 und 32. Diese sind wie bei dem Messaufnehmer nach Fig. 2 bei dem ersten Anschlussteil 31 mit dem ruderblattseitigen Teil 10 des Ruderschafts 1 und mit dem zweiten Anschlussteil mit dem rudergriffseitigen Teil 11 verbunden. Dafür ist wie in Fig. 2 die ruderblattseitige Aufnahme 12 zur Aufnahme des ersten Anschlussteils 31 und eine rudergriffseitige Aufnahme 13 zur Aufnahme eines zweiten Anschlussteils 32 vorgesehen. Die Verbindung kann eine Schraubverbindung, eine Klebeverbindung oder eine Formschlussverbindung durch ein Einschnappen sein. Vorteilhafterweise ist sie lösbar.

Vorteilhafterweise sind radiale Rillen (oder auch Rippen) 72 vorgesehen, um den Mitteilteil lösbar mit einer aufklipsbaren Abdeckung zu versehen, um die Elektronik in der Ausnehmung 74 bzw. den Durchbrüchen 78 zu schützen, die nachfolgend beschrieben werden.

Die Messaufnahme 73 weist im Mittelteil 70 am Umfang mindestens eine radiale Ausnehmung 74 zur Aufnahme des mindestens einen Dehnungsmessstreifens 4 auf. Bei der radialen Ausnehmung 74 handelt es sich um ein im Querschnitt ovales Loch, welches zudem an seiner Mündung eine Abflachung 77 des Mittelteils 70 aufweist, die ein höheres herausragendes freies Ende eines in die Ausnehmung gesteckten Bauteils ermöglicht, was die Funktion einer da vorgesehenen Antenne einer in der Figur nicht dargestellten gedruckten Schaltung in Form einer Steckplatte stark verbessert. Die gedruckte Schaltung ist ein Rechteck und hat zwei gegenüberliegende Seitenkanten. Diese haben einen vorbestimmten Anstand voneinander, der dem Abstand von zwei gegenüberliegenden Rillen 71 in den kürzeren Endflächen des im Querschnitt ovalen oder rechteckigen Querschnitts der Ausnehmung 74 entspricht, so dass die gedruckte Schaltung in einem Reibschluss zwischen die Rillen 71 einsetzbar ist. Zwei Dehnungsmessstreifen werden links und rechts neben der Ausnehmung 74 auf der Abflachung 77 befestigt.

Zwei weitere Dehnungsmessstreifen werden nebeneinander auf der abgeflachten Fläche 79 befestigt, insbesondere nebeneinander oberhalb der Öffnungen 78, in die dann die Verbindungskabel eingesteckt werden können. Prinzipiell wäre das auch über eine einzige Öffnung möglich oder um das Mittelteil 70 herum.

Die radiale Ausnehmung 74 ist im wesentlichen ein Sackloch, "im wesentlichen" deshalb, weil hier in der Tiefe der über die Mitte des Elementes mindestens ein Durchbruch 78, hier sind es zwei ovale oder rechteckige Durchbrüche 78, vorgesehen ist/sind, insbesondere an zwei gegenüberliegenden Positionen zur radialen Verlängerung der Ausnehmung 74, so dass zwei an der gedruckten Schaltung beidseitig vorgesehene Stecker in diese parallel angeordneten Durchbrüche 78 ragen.

Für eine hier in den Zeichnungen dargestellte Messaufnahme 73 wurden aus praktischen und technischen Gründen die drei oben erwähnten Elemente. i.e. MS-Messverstärker, Sender und eine Energiequelle 60 in die Hülse integriert. Die weiter oben beschriebene Verarbeitungseinheit 50 weist ferner ein mikroelektromechanisches System 6 auf, welches ausgebildet ist, um die Rotationbewegung des Ruders zu messen und ein entsprechendes Signal drahtlos an einen Empfänger 61, wie in Fig. 1 dargestellt, zu übermitteln.

Die Fig. 5 und 6 zeigt die vier wesentlichen Merkmale des hier beschriebenen Ausführungsbeispiels. Der mechanische Teil besteht aus den Elementen des Messbalkens, auf welchen vier DMS geklebt werden und in welchem ein zentraler Ausschnitt die oben erwähnten Elemente 5, 6 und 60 empfangen werden, einem Adapter 31 mit Gewinde für die Verbindung mit dem rudergriffseitigen Teil und einem weiteren Adapter 32 mit Gewinde für die Verbindung mit dem ruderblattseitigen Teil.

Das Print Circuit Board (PCB), auf welchem die oben erwähnten Elemente 5 und 6 vorhanden sind, und die Energiequelle 60 sind im Bild der Fig. 5 nicht dargestellt. Die Energiequelle 60 kann insbesondere als flacher Akkumulator neben dem PCB in den Ausschnitt 74 geschoben werden. Dabei ist dann eine Kabelverbindung zwischen Akkumulator und dem PCB vorgesehen.

Die Messaufnahme hat im Gegensatz zum Ausführungsbeispiel nach Fig. 2 und 3 keine zentrale axiale Bohrung mehr. Die Abflachungen für die Aufnahme der DMS sind in Bezug auf die zur Symmetrieebene senkrechte Mittelebene nicht identisch. Die obere Abflachung wurde tiefer ausgeschnitten, um Freiraum für eine in diesen Raum ragende Senderantenne auf dem PCB zuzulassen, neben der verbesserten Handhabbarkeit.

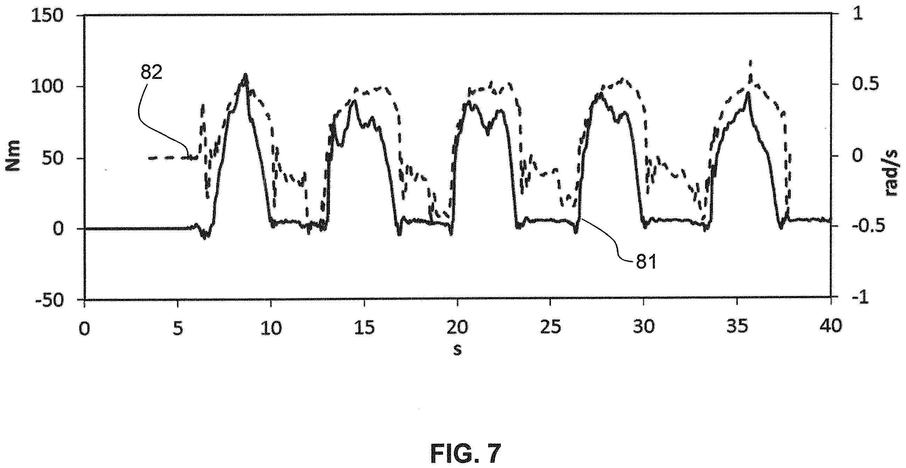

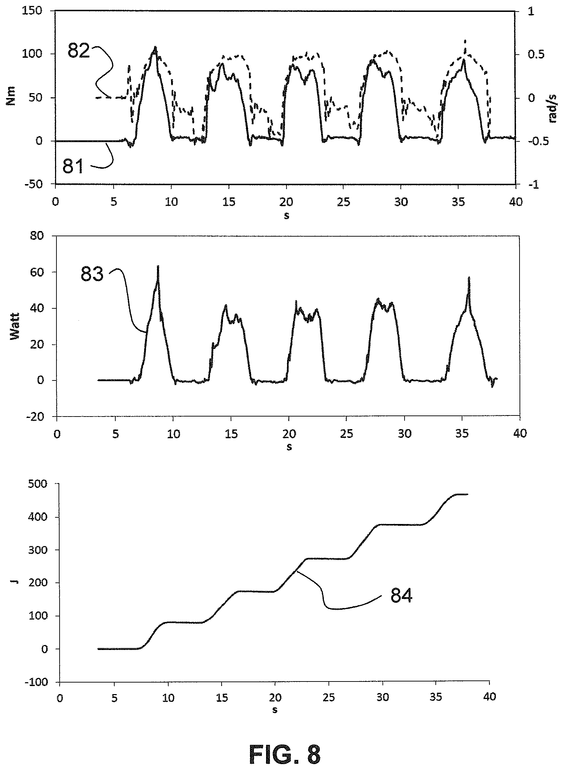

Die Fig. 7 zeigt Messkurven von Moment 81 und Rotationsgeschwindigkeit 82 eines Ruders im praktischen Betrieb einer Messaufnahme nach Fig. 5, und die Fig. 8 zeigt eine Zusammenschau einer verkleinerten Version der Fig. 7 mit sich aus einer Auswertung ergebenden Werten von Leistung 83 und geleisteter Arbeit 84 über die dargestellten fünf Ruderschläge. Fig. 7 zeigt mit der ausgezogenen Linie 81 das gemessene Moment über fünf Ruderschläge in Nm mit Peakwerten von ca. 100 Nm. Die gemessene Rotationsgeschwindigkeit liegt für die gestrichelte Kurve 82 gemäss der rechts eingetragenen Achse zwischen +- 0.5 rad/s. Die Schlagzahl lag bei ca. 12.

Die Fig. 8 nimmt die gemessenen Werte der Fig. 7 wieder auf. Diese werden in der obersten der drei Diagramme dargestellt. Darunter wird im gleichen Massstab der aus diesen Werten berechnete Wert der momentanen Leistung 83 dargestellt, bei es Peakwerte zwischen 40 und 60 Watt. Dieser Wert ist natürlich bei dem Rückholen des Ruders nicht ganz Null, aber der wesentliche Anteil sind die fünf Ausschläge, die sich beim Durchzug des Ruderblatts durch das Wasser ergeben und dabei aus einem Produkt der beiden Werte des Moments 81 und der Rotationsgeschwindigkeit 82. Die unterste Kurve stellt dann die aufsummierte Arbeit dar, die sich bei jedem Ruderschlag von einem vorherigen Plateauwert auf einen neuen Plateauwert ändert, hier ca. 100 Joule pro Ruderschlag.

## BEZUGSZEICHENLISTE

| | | | |
|---|---|---|---|
| 1 | Ruderschaft | 3 | Messhülse |
| 2 | Ruderblatt | 4 | Dehnungsmesssstreifen |
| | (DMS) | 38 | zweite Auflagefläche |
| 5 | D | 39 | radiale Bohrung |
| 6 | mikroelektromechanisches System (MEMS) | 40 | Kabel |
| | | 50 | Verarbeitungseinheit |
| 7 | Manschette | 60 | Energiequelle |
| 8 | Ring | 61 | Empfänger |
| 9 | Rudergriff | 70 | Mittelteil |
| 10 | ruderblattseitiger Teil | 71 | Rille |
| 11 | rudergriffseitiger Teil | 72 | Rille |
| 12 | ruderblattseitige Aufnahme | 73 | Messaufnahme |
| 13 | rudergriffseitige Aufnahme | 74 | Ausnehmung |
| 30 | Mittelteil | 77 | Abflachung |
| 31 | erstes Anschlussteil | 78 | Durchbruch |
| 32 | zweites Anschlussteil | 79 | Abflachung |
| 33 | Ausnehmung | 81 | Kurve des Biegemoments |
| 34 | Ausnehmung | 82 | Kurve der Rotationsgeschwindigkeit |
| 35 | zentrische Bohrung | | |
| 36 | Mittelachse | 83 | Kurve der Leistung |
| 37 | erste Auflagefläche | 84 | Kurve der geleisteten Arbeit |

**Patentansprüche**

1.   System zur Bestimmung eines Biegemoments an einem Ruder während eines Ruderschlags eines Ruderers im Wasser aufweisend:

ein Rudergriff (9);

ein Ruderschaft (1), welcher sich vom Rudergriff (9) erstreckt;

ein Ruderblatt (2), welches am Ruderschaft (1) auf der dem Rudergriff (9) gegenüberliegenden Seite angeordnet ist;

mindestens ein Dehnungsmessstreifen (4), aus dessen Signal sich das Biegemoment bestimmen lässt;

**dadurch gekennzeichnet, dass**

der Ruderschaft (1) mehrteilig ausgebildet ist, dass zwischen zwei benachbarten Teilen des Ruderschafts (1) eine Messaufnahme (3; 73) angeordnet ist, welche die beiden benachbarten Teile des Ruderschafts (1) miteinander verbindet und welche den mindestens einen Dehnungsmessstreifen (4) aufweist, dass ein mikroelektromechanisches Systems (6) am Ruder derart angeordnet ist, um die Rotationsbewegung des Ruders um eine Achse parallel zum Biegemomentvektor über die Zeit zu ermitteln, und dass eine Auswerteschaltung vorgesehen ist, um die Ruderleistung über die Zeit aus dem skalaren Produkt des Biegemoments und der Rotationsbewegung zu bestimmen.

2. System nach Anspruch 1, wobei der mindestens eine Dehnungsmessstreifen (4) an der Oberfläche der Messaufnahme (3; 73) angeordnet ist.

3. System nach einem der Ansprüche 1 oder 2, wobei die Messaufnahme (3; 73) zylindrisch mit einer Mittelachse (36) ausgebildet ist und einen Mittelteil (30) und zwei Anschlussteile (31, 32) zur Verbindung mit den beiden benachbarten Teilen des Ruderschafts (1) aufweist, wobei die Anschlussteile (31, 32) auf sich gegenüberliegenden Seiten des Mittelteils (30; 70) angeordnet sind.

4. System nach Anspruch 3, wobei die beiden Anschlussteile (31, 32) bezüglich des Mittelteils (30; 70) abgesetzt sind und der Mittelteil (30; 70) einen Anschlag für die beiden benachbarten Teile des Ruderschafts (1) bildet, wobei optional das Mittelteil (30; 70) am Anschlag durch ein Gewinde fixiert ist.

5. System nach Anspruch 3 oder 4, wobei alternativ die Messaufnahme (3; 73) im Mittelteil (30; 70) am Umfang mindestens eine Ausnehmung (33, 34; 77, 79) zur Aufnahme des mindestens einen Dehnungsmessstreifens (4) aufweist, oder die Messaufnahme (3; 73) im Mittelteil (30; 70) in Richtung der Mittelachse (36) am äusseren Umfang zwei sich gegenüberliegende Ausnehmungen (33, 34, 77, 79) zur Aufnahme von jeweils einem Dehnungsmessstreifen (4) aufweist.

6. System nach Anspruch 5, wobei die Messaufnahme (73) einen in longitudinaler Richtung der Messaufnahme (73) ausgerichteten, radialen Schlitz (74) aufweist, in den eine mit dem oder den Dehnungsmessstreifen (4) verbundene Schaltung (50) eingeschoben ist, wobei optional der Schlitz (74) mindestens eine durchgehende Öffnung (78) aufweist, um die Schaltung mit einem oder mehreren nahe der Öffnung (78) angeordneten Dehnungsmessstreifen (4) zu verbinden und wobei eine Energiequelle (60) neben der Schaltung (50) in den Schlitz (74) eingeschoben ist.

7. System nach einem der Ansprüche 1 bis 6, wobei die Messaufnahme (3) eine zentrische Bohrung (35) aufweist, welche sich entlang der Mittelachse (36) durch die gesamte Messaufnahme (3) erstreckt, wobei optional der mindestens eine Dehnungsmessstreifen (4) an der inneren Oberfläche der zentrischen Bohrung (35) angeordnet ist.

8. System nach einem der Ansprüche 1 bis 7, wobei alternativ der Ruderschaft (1) zweiteilig ist und in einen ruderblattseitigen Teil (10) und einen rudergriffseitigen Teil (11) aufgeteilt ist, wobei der ruderblattseitige Teil (10) an seinem dem Ruderblatt (2) gegenüberliegenden Ende eine Manschette (7) mit einem Ring (8) zur Befestigung des Ruders an einem Ruderboot aufweist und wobei die Messaufnahme (3; 73) derart ausgelegt ist, um anschliessend an die Manschette (7) mit dem ruderblattseitigen Teil (10) verbindbar zu sein; oder wobei der Ruderschaft (1) zweiteilig ist und in einen ruderblattseitigen Teil (10) und einen rudergriffseitigen Teil (11) aufgeteilt ist, wobei der rudergriffseitige Teil (11) an seinem dem Rudergriff (9) gegenüberliegenden Ende eine Manschette (7) mit einem Ring (8) zur Befestigung des Ruders an einem Ruderboot aufweist und wobei die Messaufnahme (3; 73) derart ausgelegt ist, um anschliessend an die Manschette (7) mit dem rudergriffseitigen Teil (10) verbindbar zu sein.

9. System nach einem der Ansprüche 1 bis 8, wobei die Messaufnahme (3; 73) in die beiden benachbarten Teile des Ruderschafts (1) eingeklebt ist.

10. System nach einem der Ansprüche 1 bis 9, ferner aufweisend eine Verarbeitungseinheit (50), welche durch Kabel (40) mit dem mindestens einen Dehnungsmessstreifen (4) elektrisch leitend verbunden ist; mindestens eine Energiequelle (60), welche mit dem mindestens einen Dehnungsmessstreifen (4) und der Verarbeitungseinheit (50) elektrisch leitend verbunden ist, um diese mit elektrischer Energie zu versorgen; wobei die Verarbeitungseinheit (50) einen DMS-Verstärker (5) aufweist, welcher ausgebildet ist, um ein von dem mindestens einen Dehnungsmessstreifen (4) erzeugtes Signal zu verstärken und drahtlos an mindestens einen Empfänger (61) zu übermitteln.

11. System nach Anspruch 10, wobei die Verarbeitungseinheit (50) ein mikroelektromechanisches System (6) aufweist, welches ausgebildet ist, um die Rotationbewegung des Ruders zu messen und ein entsprechendes Signal drahtlos an mindestens einen Empfänger (61) zu übermitteln.

12. System nach Anspruch 10 oder 11, wobei alternativ die Verarbeitungseinheit (50) im rudergriffseitigen Teil (11) des Ruderschafts (1) angeordnet ist; oder wobei die Verarbeitungseinheit (50) im ruderblattseitigen Teil (10) des Ruderschafts (1) angeordnet ist.

13. System nach einem der Ansprüche 10 bis 12, wobei der mindestens eine Empfänger (61) ein Desktop, ein Laptop, ein Tablet, eine Smartwatch oder ein Smartphone ist oder wobei der mindestens eine Empfänger (61) eine Einheit ist, beispielsweise ein USB-Stick, welche mit einem Desktop, einem Laptop, einem Tablet oder einem Smartphone verbindbar ist.

14. Verfahren zur Bestimmung einer Leistung an einem Ruder während eines Ruderschlags eines Ruderers im Wasser mittels eines System nach einem der vorhergehenden Ansprüche 1 bis 13, aufweisend die folgenden Schritte:

Ermitteln des Biegemoments am Ruder über die Zeit mittels des mindestens einen Dehnungsmessstreifens (4) Ermitteln der Rotationsbewegung des Ruders um eine Achse parallel zum Biegemomentvektor über die Zeit mittels des mikroelektromechanischen Systems (6); und Ermitteln der Ruderleistung über die Zeit aus dem Produkt des Biegemoments und der Rotationsbewegung.

**Claims**

1. System for determining a bending moment on an oar during a rowing stroke in the water comprising:

an oar handle (9);
an oar shaft (1) that extends from the oar handle (9); an oar blade (2) that is disposed on the oar shaft on a side opposite to the oar handle (9);
at least one strain gauge (4), whose signal determines the bending moment;
**characterized by** the fact
that the oar shaft is constituted in different parts, that between two adjacent parts of the oar shaft (1) a measuring transducer (3; 73) is located, which connects the two adjacent parts of the oar shaft (1) and which has at least one strain gauge (4), that a microelectromechanical system (6) is located in order to measure over the time the rotation speed of the oar around an axis parallel to the bending moment vector and that an analysis circuit is foreseen in order to determine the rowing power over the time through the scalar product of the bending moment and the rotation speed.

2. The system according to claim 1, wherein at least one strain gauge (4) is located on the surface of the measuring transducer (3; 73).

3. The system according to claim 1 or 2, wherein the measuring transducer (3; 73) has essentially a cylindrical shape with a central axis (36) and comprises a central part (30) und two connecting parts (31, 32) for connecting to the two adjacent parts of the oar shaft (1), wherein the connecting parts (31, 32), are disposed on mutually opposite sides of the central part (30; 70).

4. The system according to claim 3 wherein the two connecting parts (31, 32) are offset in relation to the central part (30; 70) and the central part (30; 70) forms a stop for the two of the at least two adjacent parts of the oar shaft (1), wherein optionally the central part (30; 70) is fixed to the stop by a thread.

5. System according to claim 3 or 4 wherein alternatively the measuring transducer (3; 73) in the central part (30; 70) on an outer periphery comprises at least one recess (33, 34; 77 ; 79) for accommodating at least one strain gauge (4), or the measuring transducer (3; 73) in the central part (30; 70) comprises in a direction of the central axis (36) on an outer periphery two mutually opposite recesses (33, 34; 77 ; 79) each for accommodating at least one strain gauge (4).

6. System according to claim 5, wherein the measuring transducer (73) comprises a radial slot (74) oriented in longitudinal direction of the measuring transducer (73), in which an analysis circuit (50) connected to the stain gauge or strain gauges is located, wherein optionally the slot (74) comprises at least one through opening (78), in order to connect the analysis circuit with one ore many strain gauges located close to the through opening (78) and wherein an energy source (60) is inserted into the radial slot (74) next to the analysis circuit (50).

7. System according to claims 1 to 6, wherein the measuring transducer (3) comprises a central bore (35), which extends along the central axis through all the measuring transducer (3), wherein optionally at least one strain gauge (4) is disposed on an inner surface of the central bore (35).

8. System according to one of the claims 1 to 7, wherein alternatively the oar shaft (1) is in two parts and is divided into a part on a side of the oar blade (10) and a part on a side of the oar handle (11), wherein the part on the side of the oar blade (10) comprises a collar (7) on an end opposite to the oar blade (2) with a ring for attaching the oar to a rowing boat, wherein the measuring transducer (3; 73) is subsequently joined to the collar (7) with the part on the side of the oar blade (1), or wherein the oar shaft (1) is in two parts and is divided into a part on aside of the oar blade (10) and a part on a side of the oar handle (11), wherein the part on the side of the oar handle (11)on the end thereof opposite the oar handle (9) comprises a collar (7) with a ring (8)for attaching the oar to the rowing boat and wherein the measuring transducer (3; 73) is subsequently joined to the collar (7) with the part on the side of the oar handle (11).

9. System according to claims 1 to 8, wherever the measuring transducer (3; 73) is glued into the two adjacent parts of the oar shaft (1).

10. System according to claims 1 to 9 which comprises farther a processing unit (50), that is electrically conductively connected by cable at the at least one strain gauge (4), at least one energy source (60), that is electrically conductively to the at least one strain gauge (4) and to the processing unit (50) in order to supply said at least one strain gauge (4) and processing unit (50) with electrical energy; wherein the processing unit (50) comprises a strain gauge amplifier (5) that is designed to amplify a signal that is produced by the at least one strain gauge (4) and to transmit said signal wirelessly to the at least one receiver (61).

11. System according to claim 10, wherein the processing unit (50) comprises the microelectromechanical system (6) that is designed to measure the rotational motion of the oar and to transmit a corresponding signal wirelessly to at least one receiver (61).

12. System according to claim 10 or 11, wherein alternatively the processing unit (50) is disposed in the part of the oar shaft (1) on the oar handle side, or the processing unit (50) is disposed in the part of the oar shaft (1) on the oar blade side.

13. System according to the claims 10 to12, wherein at least one receiver (61) is a desktop, a laptop, a tablet, a smartwatch or a smartphone or wherein at least one receiver (61) is a unit, for example a USB Stick, which can be connected to a desktop, a laptop, a tablet or a smartphone.

14. A method for determining the power on an oar during a rowing stroke in the water, comprising the following steps:

determining the bending moment on the oar against time by mean of at least one strain gage that is disposed on the measuring transducer (3) that is disposed between two adjacent parts of an oar shaft (1) of the oar and that connects said adjacent parts to each other;
determining the rotation rate against time of the oar about an axis parallel to a bending moment vector by a microelectromechanical system, which is disposed on the oar;
and determining the rowing power against time from the scalar product of the bending moment and the rotation rate.

**EP 3 187 849 B1**

## Revendications

1. Système destiné à déterminer le moment de flexion de l'aviron pendant un coup d'aviron dans l'eau comportant;
une poignée d'aviron (9) ;
une hampe d'aviron (1), lequel prolonge le poignée d'aviron (9) ;
une pelle d'aviron (2), laquelle se trouve sur la hampe d'aviron (1) à l'extrémité opposée à la poignée d'aviron (9) ;
au moins une jauge de contrainte (4), dont le signal permet de déterminer le moment de flexion ;
**caractérisé par le fait que** la hampe d'aviron (1) est composée de plusieurs parties, qu'entre deux parties adjacentes de la hampe d'aviron se trouve le manchon de mesure (3 ; 73), lequel relie les deux parties adjacentes de la hampe d'aviron (1) et lequel comporte au moins une jauge de contrainte (4), qu'un système électromécanique miniature (6) est placé sur l'aviron de sorte qu'il mesure la vitesse de rotation de l'aviron en fonction du temps selon un axe parallèle à l'axe du moment de flexion et qu'un circuit électronique est prévu pour calculer la puissance en fonction du temps à partir du produit scalaire du moment de flexion avec la vitesse de rotation.

2. Système selon la revendication 1, dans lequel au moins une jauge de contrainte (4) se trouve à la surface du manchon de mesure (3 ; 73).

3. Système selon l'une des revendications 1 ou 2, dans lequel le manchon de mesure (3 : 73) est de forme essentiellement cylindrique avec un axe central (36), et comportant une partie centrale (30) et deux éléments de raccordement (31, 32) pour le raccord avec les deux parties adjacentes de la hampe d'aviron (1), dans lequel les éléments de raccordement (31, 32) se trouvent aux deux extrémités opposées de la partie centrale (30; 70).

4. Système selon la revendication 3, dans lequel les deux éléments de raccordement (31, 32) comportent un décrochement par rapport à la partie centrale (30 ; 70) et la partie centrale (30; 70) forme une butée pour les deux parties adjacentes de la hampe d'aviron, dans lequel de manière optionnelle la partie centrale (30 ; 70) est fixé par un pas de vis à l'endroit de la butée.

5. Système selon la revendication 3 ou 4, dans lequel comme solution alternative le manchon de mesure (3, 73) présente sur le pourtour de la partie centrale (30; 70) au moins une encoche (33, 34 ; 77 ; 79) pour le placement d'au moins une jauge de contrainte (4), ou bien dans lequel le manchon de mesure (3, 73) présente dans la partie centrale (30; 70) en direction de l'axe central (36) sur le pourtour extérieur deux encoches opposées (33, 34 ; 77, 79) pour le placement dans chacune d'une jauge de contrainte (4).

6. Système selon la revendication 5, dans lequel le manchon de mesure (73) présente une fente radiale (74) disposée en direction longitudinale du manchon de mesure (73), dans laquelle est logée un circuit électronique (50) relié à la ou aux jauges de contraintes (4), dans lequel de manière optionnelle la fente (74) présente au moins une ouverture (78) passant à travers, pour connecter le circuit électronique avec une ou plusieurs jauges de contraintes (4) situées à proximité de l'ouverture (78), dans laquel une source d'énergie (60) est logée près du circuit électronique (50) dans la fente (74).

7. Système selon l'une des revendications 1 à 6, dans lequel le manchon de mesure (3) présente un perçage central (35), qui traverse le manchon de mesure (3) de part en part, dans lequel de manière optionnelle la jauge de contrainte (4) au moins au nombre de un est placée sur la surface intérieure du perçage central (35).

8. Système selon une des revendications 1 à 7, dans lequel de manière alternative la hampe d'aviron (1) est constituée de deux parties et est divisée en une partie côté pelle d'aviron (10) et une partie côté poignée d'aviron (11), dans lequel la partie côté pelle d'aviron (10) présente à son extrémité opposée à la pelle d'aviron (2) une manchette (7) et un anneau (8) destinés à relier l'aviron au bateau d'aviron et dans lequel manchon de mesure (3 ; 73) est relié directement à la partie côté pelle d'aviron (10) à l'endroit de la manchette (7), ou bien dans lequel la hampe d'aviron (1) est constituée de deux parties et est divisée en une partie côté pelle d'aviron (10) et une partie côté poignée d'aviron (11), dans lequel la partie côté poignée d'aviron (11) présente à son extrémité opposée à la poignée d'aviron (9) une manchette (7) et un anneau (8) destinés à relier l'aviron au bateau d'aviron et dans lequel manchon de mesure (3 ; 73) est relié directement à la partie côté poignée d'aviron (11) à l'endroit de la manchette (7).

9. Système selon les revendications 1 à 8, dans lequel le manchon de mesure (3 ; 73) est collé dans les deux parties adjacentes de la hampe d'aviron (1).

10. Système selon une des revendications 1 à 9, qui présente en outre un circuit électronique (50), lequel est relié électriquement par un câble conducteur (40) à au moins une jauge de contrainte (4); au moins une source d'énergie (60), laquelle est reliée électriquement de manière conductrice avec au moins l'une des jauges de contrainte (4) et avec le circuit électronique (50), afin de les alimenter en énergie électrique; dans lequel le circuit électronique (50) comporte un amplificateur de jauge de contrainte (5), lequel est conçu pour amplifier le signal issu d'au moins une jauge de contrainte (4) et pour le transmettre par onde électromagnétique à au moins un récepteur (61).

**11.** Système selon la revendication 10, dans lequel le circuit électronique (50) comporte un système électromécanique miniature (6), lequel es conçu pour mesurer le mouvement de rotation de l'aviron et pour transmettre le signal correspondant par onde électromagnétique à au moins un récepteur (61).

**12.** Système selon la revendication 10 ou 11, dans lequel comme solution alternative le circuit électronique (50) est placé dans la partie côté poignée d'aviron (11) de la hampe d'aviron (1) ; ou dans lequel le circuit électronique (50) est placé la partie côté pelle d'aviron (10) de la hampe d'aviron (1).

**13.** Système selon une des revendications 10 à 12, dans lequel le récepteur (61), au moins au nombre de un, est un desktop, un laptop, une tablette, une smartwatch ou un smartphone, ou dans lequel le récepteur (61), au moins au nombre de un, est une unité, par exemple un stick USB, lequel peut être connecté à un desktop, un laptop, une tablette ou un smartphone.

**14.** Procédure pour déterminer la puissance transmise par un aviron pendant un coup d'aviron dans l'eau d'un rameur comportant les pas suivants: détermination du moment de flexion de l'aviron en fonction du temps au moyen d'une jauge de contrainte au moins, laquelle est placée dans un manchon de mesure (3), lequel est placé entre deux partie adjacentes d'une hampe d'aviron (1) et qui relie ces deux parties ;
détermination du mouvement de rotation de l'aviron autour d'un axe parallèle au vecteur du moment de flexion en fonction du temps au moyen d'un système électromécanique miniature (6), lequel est placé dans l'aviron; et détermination de la puissance du coup d'aviron en fonction du temps par le produit du moment de flexion et du mouvement de rotation.

FIG. 1

FIG. 2

EP 3 187 849 B1

**FIG. 3**

**FIG. 4**

**FIG. 5**

**FIG. 6**

**FIG. 7**

EP 3 187 849 B1

FIG. 8

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20100167606 A1
- US 2011223816 A1
- GB 2405947 A